# EUROPEAN PATENT APPLICATION

(11) **EP 4 659 696 A1**
(43) Date of publication of application: **10.12.2025**
(21) Application number: 25181035.4
(22) Date of filing: 05.06.2025
(51) Int. Cl.: A61B 34/10, A61B 34/20, A61B 34/30, A61B 90/00, A61B 17/00

(54) **NAVIGATED DISCECTOMY AND TOOL DIFFERENTIATION**

(30) Priority: 05.06.2024 US 202418734156
(71) Applicant: Globus Medical, Inc, Audubon, PA 19403 (US)
(72) Inventor: SULLIVAN, Myles, PHILADELPHIA, 19123 (US); WEIMAN, Mark, DOWNINGTOWN, 19335 (US); TAUBENKRAUT, Carly, PERKASIE, 18944 (US); MCLAUGHLIN, Corbett, BRYN MAWR, 19010 (US)
(74) Representative: Morabito, Sara

(57) **Abstract**

Dynamic discectomy platform systems, tool differentiation systems, and related methods. A navigated discectomy instrument may have a tracking array and an articulating distal tip with a corresponding tracking marker configured to track movement of the distal tip when articulated. The discectomy instrument may be navigated using a robotic navigation platform, which views and tracks the instrument throughout the discectomy procedure. The discectomy platform may include imaging, planning, and machine learning to improve the quality and effectiveness of the discectomy. The instrumentation may be modular with automatic tool differentiation for discerning different types of articulating tips.

## Description

### FIELD OF THE INVENTION

The present disclosure relates to navigated instruments and methods of implementing navigation for medical procedures, such as a discectomy during an orthopedic spinal decompression surgery.

### BACKGROUND OF THE INVENTION

Discectomy is the surgical practice of removing the soft tissue (disc) that separates and provides cushion between vertebral bodies on a given level. The procedure varies in its level of invasiveness from surgeries aimed at alleviating the pain caused by a herniated disc impinging on an exiting nerve root, to more complex techniques involving removal of the degenerative disc to facilitate an interbody fusion procedure. Common discectomy practices rely heavily on static instrumentation that may be straight or angled with varying profiles, such as curettes, cups, scrapers, etc. These instruments may be used in open, mini-open, and larger tube procedures.

For discectomy through a small tube, the procedure may include removing the nucleus pulposus and annulus fibrosus of the disc, for example, to prepare for a lumbar fusion procedure. While the smaller tubular access window reduces the morbidity of access into the disc space, as well as a clear, protected corridor to freely pass instruments through, the tube constrains surgeon capabilities to disrupt disc material, evacuate soft tissue, and properly prepare the vertebral endplates.

The introduction of navigation and robotics into spine surgery has enhanced safety and improved efficiency for surgeons during spinal procedures. Imaging and navigation technologies combined with robotics have enabled surgeons to receive real time feedback on clinically significant parameters that previously could not be assessed intraoperatively. Tool navigation provides the ability to track the location of tools in-situ when direct visualization is not possible. There exists a need, however, for a tubular discectomy platform that pairs dynamic instrumentation with navigation and tool differentiation for various tool types and orientations.

### SUMMARY OF THE INVENTION

To meet this and other needs, dynamic discectomy platform systems, tool differentiation systems, and related methods are provided. The dynamic discectomy platform may include a modular platform to allow for surgeon procedural and preferential customization for each case. The system may leverage preoperative planning with specified instrumentation to isolate soft tissue removal. The system may help to improve the user's ability to attain a quality discectomy through quantifying completeness intraoperatively and may reduce the cognitive load required during surgery by engaging with the user preoperatively, intraoperatively, and postoperatively. The navigated tool differentiation system may include a modular platform that automates tool differentiation in the operating room. The differentiation system may help to simplify the swapping of tool attachments, for example, on an independent navigated handpiece that recognizes each tool attachment independently.

According to one embodiment, a method of performing a discectomy may include: (a) providing a database model based on existing patient data with discectomy data; (b) conducting preoperative imaging and planning for a given patient; (c) obtaining a customized surgical plan from the database model including an approach, location, and degree of discectomy for the given patient; (d) performing a discectomy based on guidance from the surgical plan and collecting intraoperative data; (e) conducting a postoperative analysis after the discectomy and collecting postoperative data; and (f) updating the database model based on the intraoperative and postoperative data.

The method of performing a discectomy may include one or more of the following features. The discectomy may be conducted with a navigated articulating discectomy instrument. For a non-fusion procedure, the discectomy may target an identified surgical level and identify soft tissue to be removed based on a volumetric heatmap. For a fusion procedure, the discectomy may target an identified surgical level, select a surgical approach, identify soft tissue to be removed based on a volumetric heatmap, and prepare the disc space for insertion of an interbody implant. The method may also include modifying the volumetric heatmap based on intended placement of the interbody implant. The database model may incorporate artificial intelligence to enhance database functionality, data analysis, and predictions. The database model may be incorporated into software of an on-board computer for a surgical robotic and navigation system.

According to one embodiment, a method of performing a spinal decompression of a patient may include: (a) obtaining a customized discectomy plan from a database model with existing discectomy data based on patient specific parameters for the patient; (b) inserting a navigated discectomy instrument into the disc space, the instrument having an articulating distal tip and a corresponding tracking marker configured to track movement of the distal tip when articulated; (c) tracking location, orientation, and movement of the articulating distal tip by a navigation system to perform a discectomy based on the customized discectomy plan; and (d) collecting data throughout the discectomy and updating the database model with the newly collected data.

The method of performing a spinal decompression may include one or more of the following features. The tracking marker may follow an arc to finely track the tip location as it articulates. A path of the tracking marker may be constant for a life cycle of the tip movement. The tracking marker may be coupled to a moveable handle, which actuates the articulating distal tip. The navigated discectomy instrument may be navigated with a tracking array having multiple orientations to maintain line of sight to the navigation system. The tracking array may be rotatable to discrete left, right, and top orientations. The method may include swapping one articulating distal tip and installing another type of articulating distal tip on the navigated discectomy instrument. The method may also include reorienting the articulating distal tip by releasing a shaft and rotating the shaft to reorient the tip 180 degrees relative to its initial position.

According to one embodiment, a system for performing a discectomy may include a surgical robotic and navigation system and a navigated discectomy instrument. The surgical robotic and navigation system may have an on-board computer with software executed by one or more processing units, and storing and executing an existing database model with existing discectomy data. The navigated discectomy instrument may have a tracking array and an articulating distal tip with a corresponding tracking marker configured to track movement of the articulating distal tip when articulated. The surgical robotic and navigation system may provide customized guidance to a surgeon during a discectomy. The location, orientation, and movement of the articulating distal tip may be trackable by the surgical robotic and navigation system. The navigated discectomy instrument may include a modular handpiece for receiving a variety of tool attachments with different types of articulating tips. The navigated discectomy instrument may include a handpiece with a barrel for receiving a shaft, a fixed handle, and a moveable handle pivotably coupled to the fixed handle configured to articulate the articulating distal tip. The barrel may include a rotatable collar configured to rotate about a longitudinal tool axis of the barrel to orient the tracking array, and the rotatable collar may include a rotational lock configured to secure and lock the tracking array into place when the array is optimally positioned. The shaft may include a depth control including a pivotable stop such that when the articulating distal tip is rotated, the stop pivots and maintains a fixed length between a distal-most point of the articulating distal tip and a distal-most point of the stop. The navigated discectomy instrument may be compatible with extended reality. The tracking array may be tilted toward the user for improved visualization, for example, on a user's headset to enhance extended reality tracking capabilities. Alternatively, the tracking markers may be removed and a machine vision system may be substituted to track the instruments.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIGS. 1A-1B depict a navigated articulating tool with a moveable tracking marker configured to track movement of the tool's articulating tip according to one embodiment;
FIGS. 2A-2B show examples of surgical robotic and navigation systems;
FIG. 3A-3B illustrate a workflow and flowchart for a machine learning discectomy model;
FIGS. 4A-4B show a navigated articulating tool with tracking markers having linear and rotary paths, respectively, to track movement of the articulating tip according to one embodiment;
FIGS. 5A-5C show perspective views of a modular, swappable array handpiece with a pilot array attached to the handpiece according to one embodiment;
FIGS. 6A-6C show side views of attachment of the pilot array to the handpiece as shown in FIGS. 5A-5C;
FIGS. 7A-7C show perspective views of rotation of the array about the rotatable collar for optimal visibility by the camera system;
FIGS. 8A-8C show swapable array positions with a moveable dual marker as compared to a single marker for tracking the articulating tip according to one embodiment;
FIGS. 9A-9D show tool attachment loading of the tool shaft into the navigated handpiece according to one embodiment;
FIGS. 10A-10C show tool attachment loading into the navigated handpiece according to an alternative embodiment;
FIGS. 11A-11B show articulation of the distal tip and corresponding movement of the moveable tracking marker according to one embodiment;
FIG. 12 shows release of the tool shaft with a release button to swap the attachment according to one embodiment;
FIGS. 13A-13C show a new attachment assembled to the navigated handpiece and corresponding movement of the moveable tracking marker according to one embodiment;
FIGS. 14A-14B show reorientation of the distal tip by releasing the shaft and rotating the attachment to reorient the tip 180° from its initial position according to one embodiment;
FIGS. 15A-15B show built-in depth controls such that the articulated tool is configured to reach a predetermined maximum depth according to one embodiment;
FIGS. 16A-16C show swappable array positions that may be compatible with extended reality according to one embodiment;
FIGS. 17A-17C show examples of swappable array positions including a left position, a right position, and an extended reality user position, respectively;
FIGS. 18A-18C show a navigated articulating tool with a translating tracking marker configured to track movement of the tool's articulating tip according to one embodiment;
FIGS. 19A-19B show an alternative navigated articulating tool with linear and rotary markers for tracking the articulating tip;
FIG. 20A-20E show an alternative handpiece with different tracking marker configurations for tool differentiation according to one embodiment; and
FIGS. 21A-21B show examples of differentiating tool tips and articulation with tracking markers omitted, for example, for a machine vision system.

### DETAILED DESCRIPTION OF THE INVENTION

Embodiments of the disclosure are generally directed to dynamic discectomy platform systems, tool differentiation systems, and related methods. A discectomy is a surgical procedure aimed at relieving pressure on spinal nerves caused by a herniated or degenerative disc. The procedure may involve the removal of all or a portion of the intervertebral disc that separates the vertebrae in the spine. During a minimally invasive surgery (MIS), a small tube may be used to provide a clear, protected pathway to access the disc space. This restricted access, however, may limit the surgeon's ability to manipulate the instruments, remove the tissue, and adequately prepare the vertebral endplates.

In one embodiment, a tubular discectomy platform pairs articulating (dynamic) instrumentation with navigation to improve the quality and effectiveness of the discectomy. The discectomy instrumentation may include a dynamic or articulating tip, which enhances the precision and flexibility of disc material removal during spinal procedures. The articulating tip may bend and/or rotate to allow the surgeon to adjust the angle or approach and navigate around anatomical structures for better access to the disc. The discectomy instrument may be incorporated into computer-assisted technology platforms, such as robotic and/or navigation systems to further assist the surgeon throughout the surgical procedure. For example, the discectomy instrument may be navigated using a robotic navigation platform, which views and tracks the instrument during the discectomy procedure.

In order to further optimize the discectomy, the dynamic discectomy platform may include imaging, planning, and machine learning. For example, the system may incorporate an artificial intelligence (AI) model that offers customized recommendations to the surgeon. The system may include an in-depth preoperative analysis and plan of soft tissue removal that breaks down specific areas of discectomy. The system may navigate, catalog, and differentiate different surgical instruments. The platform may further include depth controls to enhance surgical precision and safety to ensure the tools do not penetrate deeper than necessary during the procedure. The platform may also be compatible with extended reality (XR), which may allow the surgeon to interact with preoperative plans, assess intraoperative navigation, and alter plans based on intraoperative feedback. Alternatively, the platform may be configured for use with a machine vision system to track the instrument, without markers, throughout the discectomy procedure.

In one embodiment, the instrument may be part of a modular instrument platform that automates tool differentiation in the operating room. For example, several tool attachments may utilize the same primary array pattern to recognize tool location and orientation. The modular instrument may simplifying the swapping of tool attachments on an independent navigated handpiece that recognizes each tool attachment independently. Differentiating tools autonomously may help to reduce the cognitive load on the user. The modular instrument platform may also help to reduce manual inputs and improve the user interface of navigation systems.

Although the platform and navigated instruments are generally described for performing a discectomy, it will be readily appreciated by those skilled in the art that the systems and methods described herein may be employed in any number of suitable orthopedic applications or other surgical procedures. In particular, it will be appreciated that the discectomy instrument may be modified for insertion of an articulating spacer or implant. Examples of articulating spacers or implants are described in U.S. Patent Nos. 9,770,343 and 10,765,528, which are incorporated by reference herein in their entireties for all purposes. The navigated instruments may further be configured to perform other surgical tasks needing precise movements and adjustments.

Additional aspects, advantages and/or other features of example embodiments of the invention will become apparent in view of the following detailed description. It should be apparent to those skilled in the art that the described embodiments provided herein are merely exemplary and illustrative and not limiting. Numerous embodiments or modifications thereof are contemplated as falling within the scope of this disclosure and equivalents thereto.

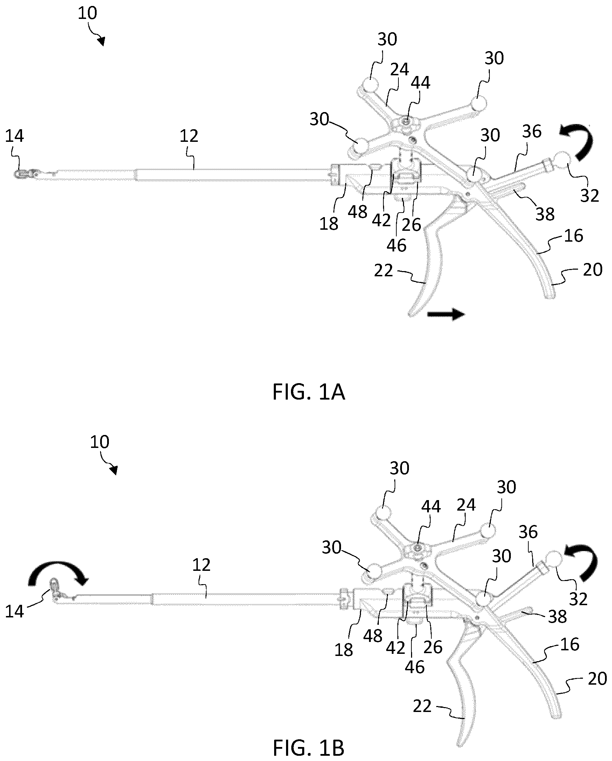

Turning now to the drawing, where like numerals may indicate like elements throughout, FIGS. 1A-1B depict a navigated articulating discectomy instrument 10 according to one embodiment. The discectomy instrument 10 may be configured to remove disc material, prepare vertebral endplates, and/or prepare an intervertebral space, for example, for insertion of a stabilizing spacer or implant, which is configured to be inserted between adjacent vertebral bodies to facilitate separation and promote fusion. Accessing the disc space during spinal surgery may be accomplished through various surgical approaches, such as anterior, lateral, transforaminal, or posterior approaches. The specific approach may be chosen, for example, based on the specific location of the disc pathology, anatomical considerations, the health of the patient, and surgical objectives including minimizing operative risks and maximizing recovery outcomes.

The discectomy instrument 10 may include a shaft 12 with an articulating distal tip 14, and the shaft 12 may be attached to a handpiece 16 configured to actuate the distal tip 14. The handpiece 16 may include a barrel 18 for receiving the shaft 12, a fixed handle 20, and a moveable handle 22. FIG. 1A shows the instrument 10 in an insertion position with the articulating distal tip 14 aligned along a longitudinal tool axis of the shaft 12 and barrel 18. When the moveable handle 22 is pulled proximally, the distal tip 14 articulates, thereby enabling a large angle of articulation of the distal tip 14. The tip 14 may be configured to articulate, for example, from 0 to 90° for more restricted maneuvers, or may bend up to 180° for a comprehensive range of motion. The range of motion may be selected depending on the type of articulating tip 14. FIG. 1B shows the instrument 10 in an articulated position with the distal tip 14 angled relative to the tool axis. The distal tip 14 may be configured for cutting or removing disc and may include, for example, a cutter, curette (e.g., cup or ring curette), rake, scalpel, pincher, grasper, scissors, or other articulating tool. Examples of articulating cutting tools are described in more detail, for example, in U.S. Patent No. 11,234,716, which is incorporated by reference herein in its entirety for all purposes. Alternatively, or in addition, the articulating tip 14 may include a light source, dilator, clamp, hose, retractor, sensor, probe, or other device needing precise control and positioning. The tip 14 may be tailored to suit its operational needs, from minimal angular adjustments in tight spaces to wide rotations for extensive reach and manipulation within the surgical site, or other functions as needed for the surgical task.

The discectomy instrument 10 may be navigated using a tracking array 24 attached to a rotatable collar 26 on the barrel 18 of the handpiece 16. The tracking array 24 may include a pattern of tracking markers 30, which identifies the instrument 10 and tracks the instrument 10 in real time. Navigation systems may utilize camera systems to locate instrumentation by detecting patterns of fiducial markers 30 found on the instruments connected to the array 24. These array patterns relate to specific coordinates that when identified may help with tool differentiating, tool location, and tool orientation. These array patterns may be found on surgical instrumentation, such as drills, taps, drivers, inserters, scrapers, etc., which track along a given linear axis.

In this embodiment, the instrument 10 includes an additional tracking marker 32 configured to track movement of the distal tip 14 when it is articulated. In this manner, the dynamic tool navigation not only recognizes a tool's location and orientation, but also tracks the tool's articulation in real time. When within view of the camera system, the instrument 10 may be detected and recognized by the pattern of the primary array 24 to determine tool location and orientation. The additional moveable marker 32 is configured to track articulation of the tip 14, for example, based on the additional marker 32 translating along a given path, such as an arc.

The additional tracking marker 32 may be attached to or an extension of the moveable handle 22, for example, via a post 36. The additional tracking marker 32 may be configured to translate or rotate along a given path, which may directly correspond to the path of the articulating tip 14. For example, FIG. 1A shows the tracking marker 32 in a first position, for example, resting against a post or stop 38 in a lowered position. As handle 22 is squeezed toward fixed handle 20, the post 36 and marker 32 travel upward and away from stop 38, as shown in FIG. 1B. The tracking marker 32 travels along the path corresponding to the articulation of the distal tip 14. Thus, the tracking marker 32 may help to confirm the location and orientation of the articulating distal tip 14 throughout the procedure.

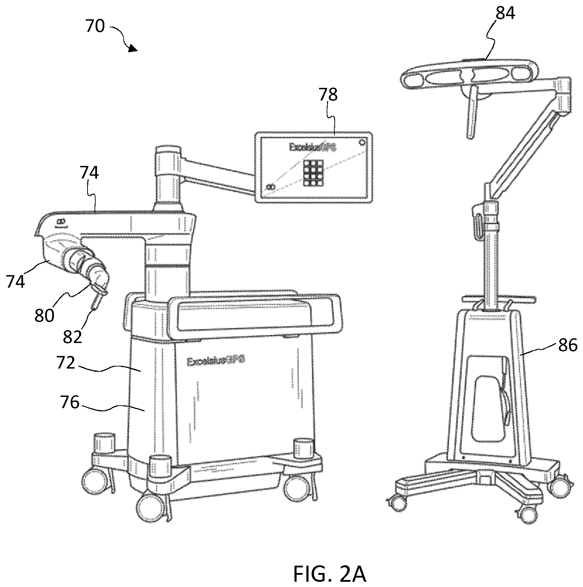

The tracking markers 30, 32 may include radiopaque or optical markers. The markers 30, 32 may be suitably shaped, including spherical, spheroid, disc, cylindrical, cube, cuboid, or the like. The tracking markers 30, 32 may be coupled to the surgical instrument in any appropriate manner. Alternatively, machine vision may be employed to track the instruments without any markers. In one embodiment, the instrument 10 may be navigated using a robotic navigation platform, which views and tracks the instrument 10 during the discectomy procedure. Examples of surgical robotic navigation systems are shown in FIGS. 2A-2B.

FIG. 2A illustrates one example of a surgical robotic navigation system 70. The surgical robot system 70 may include, for example, a surgical robot 72, one or more robot arms 74, a moveable base 76 with one or more computers having a processor, programming, and memory, a display or monitor 78 (or optional wireless tablet) electronically coupled to the computer, and an end-effector 80, for example, including a guide tube 82 electronically coupled to the computer and movable based on commands processed by the computer. The surgical robot system 70 may also utilize a camera 84, for example, positioned on a separate camera stand 86. The camera stand 86 can have any suitable configuration to move, orient, and support the camera 84 in a desired position. The camera 84 may include any suitable camera or cameras, such as one or more infrared cameras (e.g., bifocal or stereophotogrammetric cameras), able to identify, for example, active and passive tracking markers 30, 32 in a given measurement volume viewable from the perspective of the camera 84. The camera 84 may scan the given measurement volume and detect the light that comes from the markers 30, 32 in order to identify and determine the position of the markers 30, 32 in three dimensions. For example, passive markers may include retro-reflective markers that reflect infrared light (e.g., they reflect incoming IR radiation into the direction of the incoming light), for example, emitted by illuminators on the camera 84 or another suitable device.

The robotic system 72 may include one or more computer controlled robotic arms 74 to assist surgeons in planning the position of stereotaxic instruments relative to intraoperative patient images. The system 70 includes 2D & 3D imaging software that allows for preoperative planning, navigation, and guidance through a dynamic reference base, navigated instruments and positioning camera for the placement of spine, orthopedic, or other devices. Further examples of surgical robotic and/or navigation systems can be found, for example, in U.S. Patent Publication No. 2019/0021795 and U.S. Patent Publication No. 2017/0239007, which are incorporated by reference herein in their entireties for all purposes.

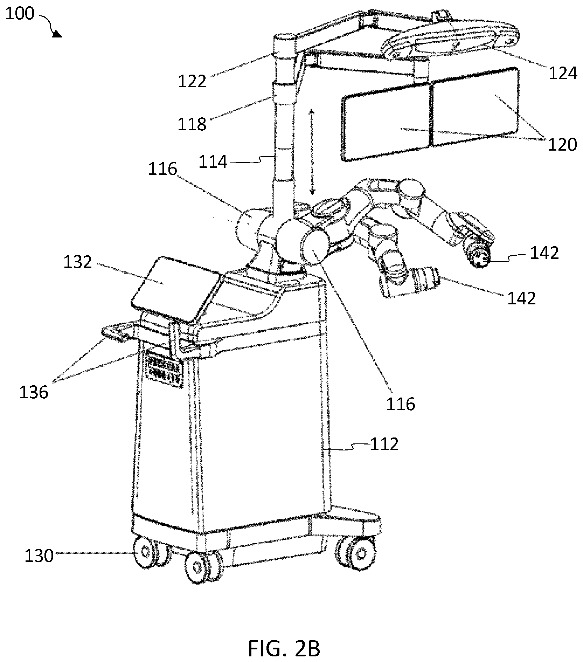

FIG. 2B illustrates another example of a surgical robotic and navigation system 100. Surgical robotic system 100 may include, for example, a moveable robotic base station 112 on wheels 130, an arm positioner 114 attached to the base station 112, and multiple arms 116, 118, 122 attached to the positioner 114. Two or more surgical arms 116 may help to guide instruments or perform surgical tasks, for example, using an end effector attachable to end effector interface 142 at the distal end of each arm 116. A monitor arm 118 is configured for supporting one or more displays or monitors 120 (e.g., a dual display). A camera arm 122 is configured for supporting one or more navigation cameras 124 and/or machine vision cameras. The base 112 may support a cabinet-mounted display or terminal 132 and includes handles 136 for transporting and positioning the system 100.

In both robotic systems 70, 100, the base station 76, 112 houses an on-board computer or computing unit for controlling all functionality of the robotic system 70, 100. The on-board computer may include a central processing unit (CPU), memory, and an input/output interface. The central processing unit carries out the instructions of a computer program or software by performing arithmetical, logical, control, and input/output (I/O) operations specified by the instructions. The memory may include volatile and non-volatile memory storage that temporarily or permanently store data and instructions that are currently in use or will be needed by the central processing unit. This may include, for example, random access memory (RAM), read-only memory (ROM), and storage devices like hard drives. It will be appreciated that tangible/non-transitory computer-readable medium comprising software code or storing instructions executable by one or more processors may be adapted, when executed on a data processing apparatus, to perform any computer method set out herein. The input/output interface allows the computer system to interact with the user, take in information, and deliver results, and may include devices such as a monitor, keyboard, mouse, network interface for internet connectivity, and so forth. Although an on-board computer is exemplified herein, it will be appreciated that the computer or one or more functions may be replaced or supplemented with external devices or systems (e.g., cloud computing).

The navigated instruments 10 include markers 30, 32, which are viewable and trackable by the navigation and/or robotic platform 70, 100. Infrared signal based position recognition systems may use passive and/or active sensors or markers for tracking the objects. In passive sensors or markers, objects to be tracked may include passive sensors, such as reflective spherical balls or discs, which are positioned at strategic locations on the object to be tracked. Infrared transmitters transmit a signal, and the reflective marker reflect the signal to aid in determining the position of the object in 3D. In active sensors or markers, the objects to be tracked include active infrared transmitters, such as light emitting diodes (LEDs), and generate their own infrared signals for 3D detection. In the embodiments shown, spherical balls may be used as the passive tracking markers 30, 32.

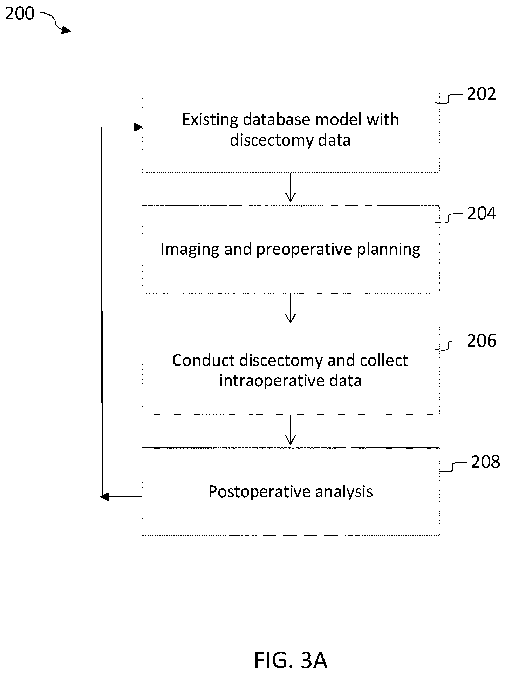

Turning now to FIGS. 3A-3B, a structured discectomy workflow may incorporate a machine learning model to help improve surgical outcomes, reduce cognitive load, and build off user specific experiences. The discectomy workflow may include imaging, pre-operative planning, intra-operative compatibility and adjustability, and post-operative analysis. FIG. 3A depicts a discectomy workflow 200 according to one embodiment.

The workflow 200 may include an existing discectomy database model 202, which is configured to store, manage, and analyze existing patient data. The database 202 may encompass various entities (e.g., tables) to capture the breadth of existing patient information, clinical data, treatment history, outcomes, and the like. The database may be comprised of inputs obtained from a variety of sources, such as literature, clinical data, publicly available data, and/or private sources, such as private hospitals, proprietary databases, or surveys. The inputs may include patient demographics, such as age, gender, weight, and general health indicators; symptoms, such as pain intensity, location, and nature of pain; diagnostic imaging, such as fluoroscopy, computed tomography (CT), magnetic resonance imaging (MRI), and X-rays; prior treatments, such as physical therapy, medications, and injections; and/or prior results following treatment, such as discectomy or fusion procedures. Any data collection practices comply with all relevant laws, such as healthcare regulations (e.g., HIPAA) and data protection regulations (e.g., GDPR).

The data points or data sets may be modeled to include averaged or normalized values to facilitate comparison and improve statistical analysis. In addition, the data may be aggregated into ranges or sets. For example, in the case of age, rather than analyzing individual ages, ages may be grouped into ranges such as 0-10 years, 11-18 years, 19-35 years, 36-50 years, 51-65 years, and older than 65. This reduces the complexity by categorizing numerous individual data points into broader, more manageable groups. The database 202 provides a robust foundation for storing, managing, and utilizing patient information, and as described in more detail below, is configured to support and guide a surgeon while performing a surgical task, such as a discectomy.

The workflow 200 may include preoperative imaging and planning 204 for a given patient. A user may access the database 202 by providing specific patient parameters for a given patient to obtain one or more outputs from the database 202. For example, the user may enter parameters, such as a patient's age, gender, and medical condition (e.g., disc herniation) to obtain outputs or a plan for the surgical procedure. The user may enter diagnostic imaging for the patient, such as MRI scans, CT scans, and X-rays to visualize the extent of the disc herniation and its impact on surrounding structures. Based on the database model 202, the system may auto-plan, for example, the approach, location, and degree of discectomy for the given patient.

The workflow 200 includes conducting the discectomy based on or with guidance from the plan and collecting intraoperative data 206 during the procedure. A step-by-step user interface may be formed from a variety of factors meant to guide and assist the healthcare professionals throughout the surgical procedure. These factors may include the degree of discectomy (quantitative data on amount removed), surgical approach, complications, tool reachability (lengths), and tool profiles and cutting orientation may be collected. This and other relevant data may be collected throughout the procedure.

The workflow may include a postoperative analysis 208 for the patient. For example, the postoperative analysis 208 may include postoperative imaging, immediate outcomes (e.g., symptom relief), recovery metrics, long-term outcomes (e.g., recurrence of symptoms or further complications), or other suitable data. The data collected during the procedure 206 and during postoperative analysis 208 may be fed back into the database model 202 to evaluate effectiveness, guide future clinical decisions, and improve patient outcomes. The workflow 200 may identify, analyze, and implement a machine learning model to serve as a foundation for continuous procedural improvement.

In one embodiment, the system may incorporate artificial intelligence (AI) to enhance database functionality with AI algorithms and machine learning (ML) models, enabling advanced data analysis and predictions. The AI algorithms may include supervised learning, unsupervised learning, semi-supervised, and reinforcement learning algorithms. The AI system may process and analyze large volumes of data, extract insights, predict trends, and learn from new data inputs over time to optimize outcomes. For example, the AI system may understand and optimize complex queries, thereby providing faster and more accurate responses. The AI system may analyze historical data to predict trends or potential pitfalls. AI-driven automation may handle routine data management tasks, such as indexing, backups, and data integrity checks. The AI system may continuously monitor its performance and automatically adjust or reorganize data to optimize performance. The AI system may identify patterns, anomalies, and correlations within the data that may not be otherwise apparent. It will be appreciated that any suitable AI algorithms or machine learning models may be used based on the most appropriate methodologies.

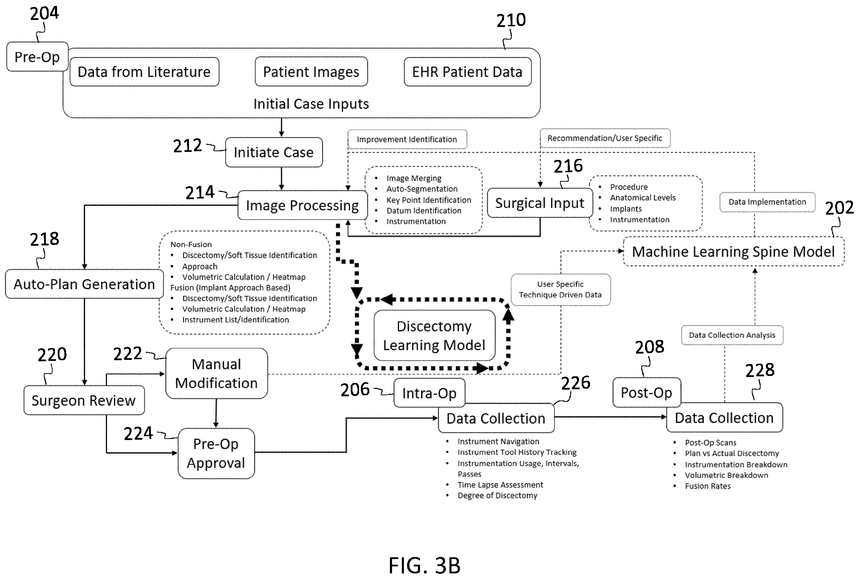

FIG. 3B depicts a more detailed flowchart of the discectomy workflow 200 including interactions with the discectomy learning model. During the preoperative planning phase 204, initial case inputs 210 may be added into the system. Initial case inputs 210 may include, for example, data from literature, patient images, and electronic health record (EHR) patient data. The user or surgeon may initiate the case 212 for a given patient. The system may complete image processing 214 with surgical input 216. The surgical input(s) 216 may include, for example, the type of procedure, anatomical levels to be corrected, implant(s) to be used, and instrumentation for the procedure. The image processing 214 may include, for example, image merging, auto-segmentation, key point identification, datum identification, and instrumentation.

From this initial information, an auto-plan 218 may be generated. The auto-plan 218 may follow one of two differing discectomy paths: a non-fusion procedure or a fusion procedure. Both paths include soft tissue disruption and evacuation but the degree of discectomy and surgical approach taken may vary greatly. For a non-fusion procedure, a non-fusion roadmap may be selected, for example, when surgery is needed to remove soft tissue that is bulging from the disc space. For example, this may occur when a herniated disc impinges on the exiting nerve root (foraminal stenosis) and/or the spinal canal (central stenosis). The planning for a non-fusion procedure may target the identified surgical level and follow a detailed checklist, for example, factoring in surgeon preference, instrument preference, volume of surgical site, surgical approach, patient anatomy, etc. The system may populate an auto-plan 218 that is based off these parameters.

In one embodiment, the auto-plan 218 may generate a volumetric heatmap that isolates the segmented disc level and soft tissue targeted for removal. The volumetric heatmap may include a simulated image of the intervertebral disc and/or disc space including access corridors, areas to target tissue removal, areas to target endplate preparation, etc. The simulated image may provide a detailed three-dimensional representation of the disc, highlighting areas to be targeted for tissue removal. The heatmap may include gradients of different colors to represent different ranges of data values (e.g., red areas requiring removal, yellow areas suggesting removal). The simulated heatmap may be manipulated and examined from various perspectives to offer insights on multiple angles and depths to assess and plan the surgery. Following generation of the auto-plan 218, the surgeon may review the plan 220, with an opportunity to manually modify the plan 222, and then provide preoperative approval 224 for the customized patient specific plan. The degree in which the plan is altered may drive machine learning implementation for that specific user in the future.

For a fusion procedure, a fusion roadmap may be selected, for example, when the goal is to fuse the adjacent vertebrae together. For example, the fusion may include bone graft, which is paired with a spacer and/or supplemental fixation to stabilize that level. The planning for a fusion procedure may target the identified surgical level and follow a detailed checklist driven off the interbody plan, for example, factoring in surgeon preference, surgical approach, patient anatomy, interbody solution, etc. The system may populate the auto-plan 218 based off these parameters and may generate a volumetric heatmap that isolates that segmented disc level and the disc material that would be within reach of the instrumentation provided. The user may manually modify the plan 222, for example, by shifting the interbody placement, which may update the generated discectomy heatmap. Once optimized, the surgeon provides preoperative approval 224 for the patient specific plan. The extent to which the plan is modified may influence the machine learning model 202 and suggestions for that particular user going forward.

Following approval, the intraoperative procedure 206 may begin. The intraoperative procedure 206 may include a discectomy to remove the damaged part of the disc, for example, based on the volumetric heatmap identified in the pre-planning. During the procedure, intraoperative data collection 226 may be stored for the discectomy learning model. The intraoperative data collection 226 may include, for example, instrument navigation, instrument tool history tracking, instrument usage, intervals, and passes, time lapse assessment, and degree of discectomy.

After the procedure is completed, a postoperative data collection 228 may occur. The postoperative data collection 228 may include, for example, post-op scans, a comparison of the plan versus actual discectomy results, instrumentation breakdown, volumetric breakdown, and fusion rates. The data from all sections may feed into the discectomy machine learning spine model 202. The model 202 may be influenced by the user specific techniques and the data collected preoperatively, intraoperatively, and postoperatively. This model 202 may then be accessed in the next patient case, for example, based on the data implementation, recommendations for a specific user, and identified improvements for the next patient. The machine learning spine model 202 may be incorporated into computer-assisted technology platforms, such as robotic and/or navigation systems 70, 100 configured to assist a user with one or more surgical tasks.

The machine learning or AI model may encapsulate patient, user, and systems data. The system may be configured to assess inputs to compare with corresponding output data to evaluate, learn, and implement techniques and recommendations customized to the surgeon (user) to quantify a quality discectomy. An in-depth preoperative analysis and plan of soft tissue removal may provide a breakdown for specific areas of discectomy. The tubular, modular discectomy platform may navigate, catalog tracking, and differentiate a variety of articulating instruments, which feed back into the AI model and relate given instrumentation to specified tasks. The platform may be customizable based on user preference and machine learning recommendations. The overall system may provide the instrumentation platform to drive improved patient outcomes while the machine learning model operates in the background to collect, assess, and implement a continuous cycle of learning.

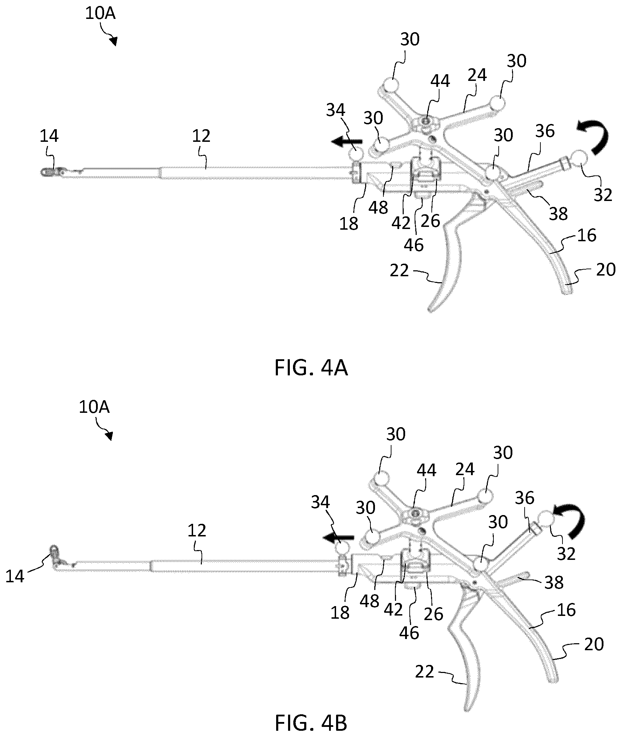

Turning now to FIGS. 4A-4B, a navigated articulating discectomy instrument 10A is shown according to one embodiment. Navigated articulating discectomy instrument 10A is similar to instrument 10 with the addition of a linear tracking marker 34. One or both of the moveable marker(s) 32, 34 can track the mechanical articulation in relation to the customized array positioning. As the user actuates the instrument 10A, the additional linear marker 34 translates forward as the rotary marker 32 pivots upward. The camera and navigation system associate the translating marker locations to the array pattern to determine the location of the articulating tip 14. In one embodiment, the fiducial or marker shape may be a sphere or a disc, for example, depending on the camera system being used and optimal design to enhance the tracking capability. The relative motion of markers 32, 34 may be used to enhance the discectomy by helping confirm tip location and orientation at all times.

The type of translating path may be linear or rotary, for example. With any type of translation, the path may be constant for the life cycle of the tip movement. As best seen in a comparison between FIGS. 4A-4B, rotary motion of marker 32 has an exponentially longer path in comparison to the linear path of linear marker 34. Thus, rotary motion may be utilized when linear translation is limited. The rotational motion causes the marker 32 to move in an arc about an axis or center of rotation, which may correlate proportionally to the articulation of the tip 14. This may enhance the camera systems ability to finely track the tip location as it articulates by providing more coordinates the fiducial 32 will pass through along its curved path. It will be appreciated that the instrument may only have a single type of translation method (e.g., rotary marker 32 or translating marker 34) for a specified attachment.

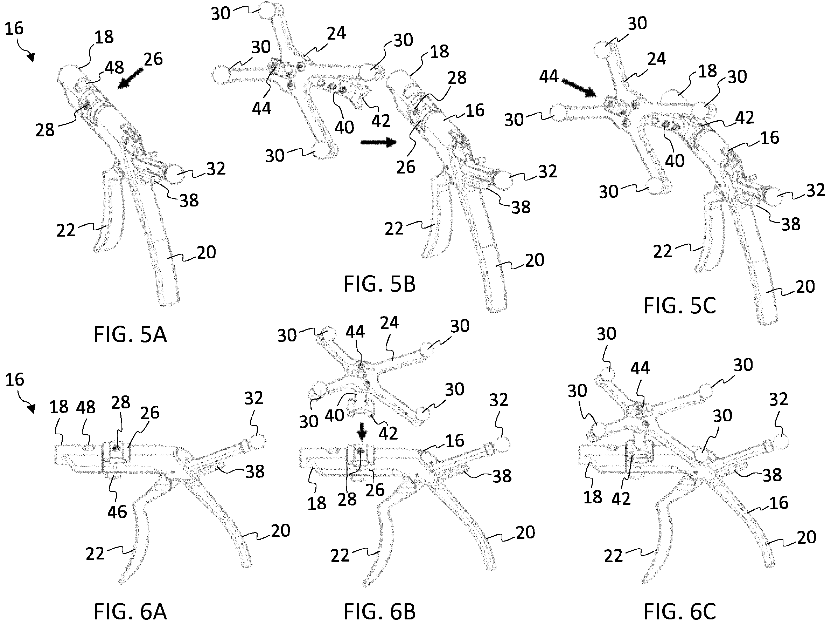

Turning now to FIGS. 5A-5C and 6A-6C, the instrument handpiece 16 may have modular and swappable capabilities, which allows the tool 10 to be used in multiple orientations to ensure any camera system can maintain a visual of the tracking markers 30 on the array 24. The barrel 18 may include a rotatable collar 26 configured to secure the array 24. The collar 26 may be configured to rotate about the longitudinal tool axis of the barrel 18 to position the array 24, for example, in left-facing, top-facing, and right-facing positions. The rotatable collar 26 may include a circular band or sleeve that fits within the barrel 18. The collar 26 may include a locking mechanism or rotational lock, such as a spring-loaded pin or ratchet system, configured to secure and lock the desired array position into place when the array 24 is optimally positioned. A plunger 46 may be engaged with the collar 26, which is configured to engage and release the rotational lock. The collar 26 may define a threaded hole 28 configured to receive a corresponding threaded set screw, bolt, or other locking feature, which secures the array 24 to the collar 26. It will be appreciated that any suitable releasable attachment mechanism may be selected.

The array 24 may include a post 40 that terminates at an attachment interface 42. The attachment interface 42 may include flanges, extensions, or protrusions at the terminating end of the post 40 configured to ensure alignment with the rotating collar 26. The flanges or protrusions may include a pair of parallel edges forming a bracket or U-shaped profile that complements corresponding slots or recesses in the rotatable collar 26. The attachment interface 42 may help to guide the post 40 into the correct position against the collar 26. Once seated onto the collar 26, a mechanical lock 44 within the array 24 may cause the threaded set screw, bolt, or other locking feature to engage the threaded hole 28 of the collar 26. For example, the mechanical lock 44 may including a shaft extending through the post 40 with a threaded tip configured to engage the threaded hole 28. When the lock 44 is rotated, the threaded set screw may be tightened in the threaded hole 28 of the collar 26, thereby securely mounting the array 24 to the collar 26. Once attached to the rotatable collar 26, the instrument 10 may be used in various orientations to guarantee that any camera system remains a clear view of the tracking markers 30 on the array 24. The modularity also offers the user the ability to operate this instrument set without the array for navigation, if desired, for example, with a machine vision system.

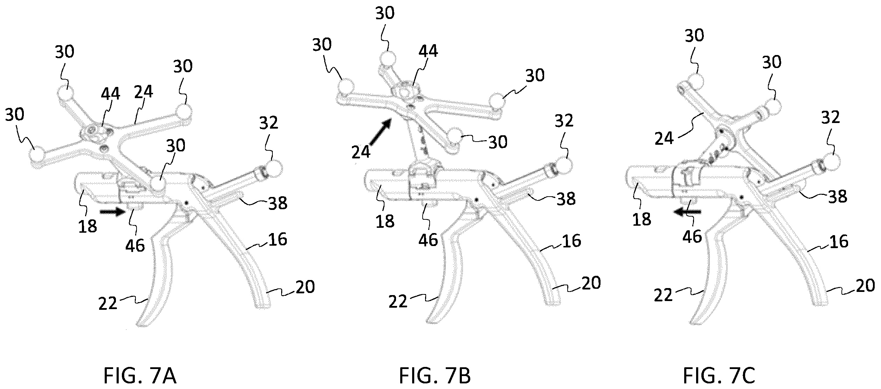

Turning now to FIGS. 7A-7C, the array 24 may be reoriented to different positions to maintain line of sight with the navigation system. Depending on the type of rotational lock, the array 24 may be locked into discrete predetermined positions, such as left, right, and top orientations, or may be continuously adjusted to any point about the range of travel of the collar 26. The connection to the rotatable collar 26 allows for the array 24 to be recognized regardless of handle position and surgeon position relative to the operating room table. In one embodiment, the array 24 may be oriented in a standard left position (shown in FIG. 7A) or a standard right position (shown in FIG. 7C). This allows visibility for the camera system from the left, right, or above the operating table. In order to adjust the array 24, the plunger 46 may be pushed back toward the fixed handle 20 to release the rotational lock (as shown by the arrow in FIG. 7A). The array post 40 may be released simply by disengaging the spring-loaded rotational lock, rotating the array 24 about the axis of the post 40 (in this case 120°), and subsequently locking the array 24 in the flipped orientation (example, right position). The collar 26 and attached array 24 may be rotated clockwise about the longitudinal tool axis of the barrel 18 (as shown in FIG. 7B). The collar 26 and attached array 24 may be rotated into the opposite orientation (as shown in FIG. 7C). The plunger 46 automatically locks into position by traveling forward away from the fixed handle 20, thereby locking the relative position of the array 24. When desired, the plunger 46 may be depressed to rotate the collar 26 and array 24 back counterclockwise to another position (as shown by the arrow in FIG. 7C). A third position may be implemented at the center of the left and right positions for a top down camera workflow (shown in FIG. 7B). It will be appreciated that the array 24 may be oriented in any suitable configuration for optimal performance.

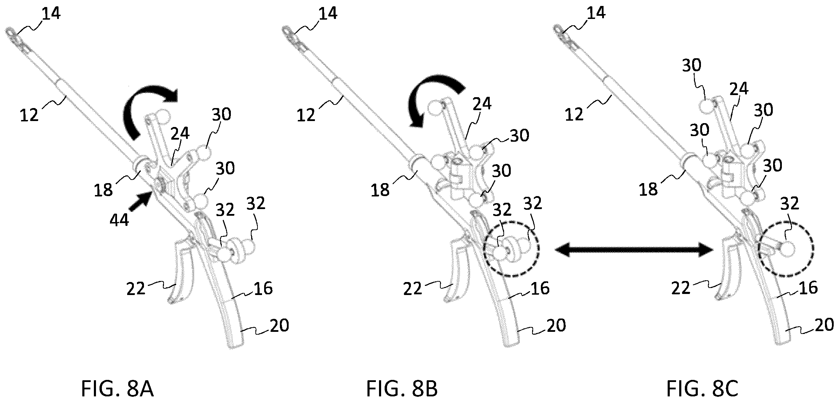

The additional, translating marker 32 may be swappable (similar to the array 24), double-sided, or singular inline to allow the tool to be actively tracked seamlessly. In one embodiment shown in FIGS. 8A-8B, the single translating marker 32 (as shown in FIG. 8C) may be replaced with double-sided markers 32 (shown in FIGS. 8A-8B). The double-sided tracking markers 32 may include two distinct spherical markers 32 on either side of a central disk. The double-sided design allows the dual markers 32 to be visible from a broad range of angles ensuring continuous tracking even if one side is obscured or facing away from the tracking system.

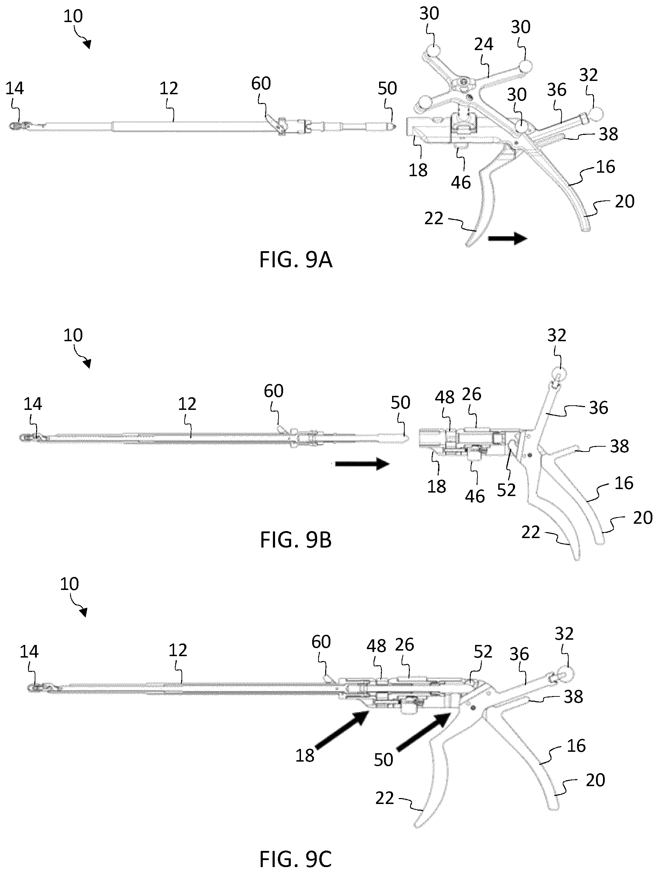

Turning now to FIGS. 9A-9D, the instrument 10 may be modular and capable of loading and unloading tool attachments onto articulating, navigated handpieces 16 with ease while maintaining a rigid connection. As best seen in FIG. 9A, the tool attachment may include tool shaft 12 having a proximal end 50 configured to seat within the barrel 18 of the handpiece 16. The proximal end 50 of the shaft 12 may be pointed or tapered to facilitate insertion. The distal end of the tool attachment 12 includes the articulating distal tip 14 suited for the particular surgical task. The tool shaft 12 may lock into the barrel 18 of the handpiece 16 via one or more locking mechanisms. In one embodiment, a button mechanism 48 may be activated by insertion of the attachment shaft 12. For example, the button mechanism 48 may include an extension, pin, bolt, or ball that protrudes into a groove or slot in the shaft 12 to securely lock it in place. When desired, the button 48 may be depressed to unlock and release the shaft 12 from the handpiece 16 for removal.

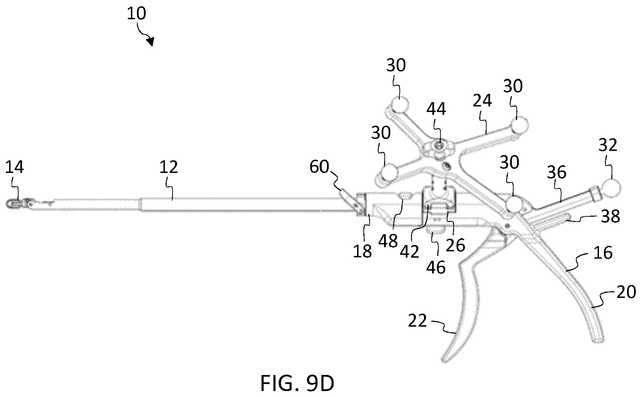

In one embodiment, the proximal end 50 of the tool shaft 12 may define an opening or notch configured to receive an extension or hook 52 on the moveable handle 22, thereby locking the shaft 12 to the handpiece 16. The hook 52 may be configured to catch onto the free end of the tool shaft 12, thereby securing the tool shaft 12 to the handpiece 16 and preventing the shaft 12 from slipping out during use. As best seen in FIG. 9B, the moveable handle 22 may form a solitary unit including the grip portion, extension post 36 for supporting the translating marker 32, and hook 52 for securing the tool shaft 12. The moveable handle 22 may be configured to pivot or rotate about a hinge or joint coupled to the fixed handle 20. When the moveable handle 22 is pulled back toward the fixed handle 20, the proximal end 50 of the tool shaft 12 may be inserted into the barrel 18 of the handpiece 16. The tool shaft 12 may be loaded from the distal end of the navigated handpiece 16, initially overextending the actuating handle 22 to align with the tool attachment 12, sliding it down the axis of the tool, and past the spring-loaded locking mechanism. Once fully seated inside the barrel 18, the moveable handle 22 may be released to allow hook 52 to pivot into engagement with the opening or notch in the proximal end 50 of the tool shaft 12, thereby securely holding it in place as best seen in FIG. 9C. The lock may automatically engage at the time the proximal end 50 of the attachment interacts fully with the actuating handle 22, and at this time, the user is free to articulate and navigate the specified tool. FIG. 9D shows the fully assembled instrument 10, which is ready for navigation and tip articulation to perform the desired surgical task.

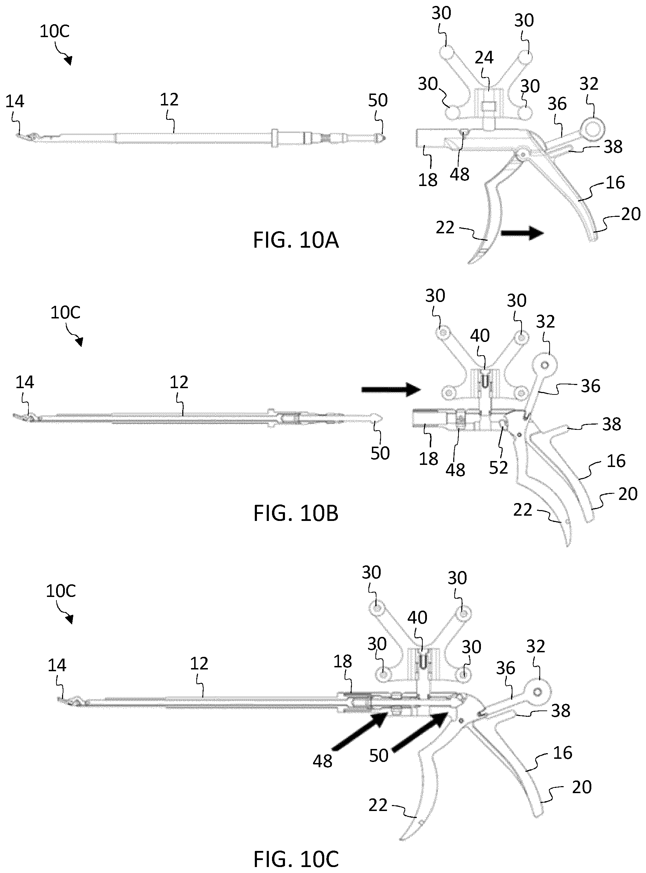

FIGS. 10A-10C depict an alternative configuration of instrument 10C for the tool attachment loading and adjustment of array 24. In the same manner as described above, the moveable handle 22 may be pulled back to force loading of the shaft 12 to the handpiece 16. The new attachment shaft 12 may be pushed into the handpiece 16. When the handle 22 is released, the tool attachment shaft 12 locks into the handpiece 16. Although a hook locking mechanism is exemplified herein, it will be appreciated that any suitable latch, engagement interface, or quick connect coupling may be used to removably couple the shaft 12 to the handpiece 16. The modular design facilitates easy attachment and detachment of the tool shaft 12 while ensuring a stable and secure connection. In addition, in this embodiment, the rotatable collar 26 is replaced with a pivotable array 24 to reposition the array markers 30. The post 40 may form or include a pivot member configured to allow for rotation of the array 24 about the post 40 such that the array 24 may be reoriented as desired by the user. The final orientation of the array 24 may be locked for optimal visibility by the navigation system.

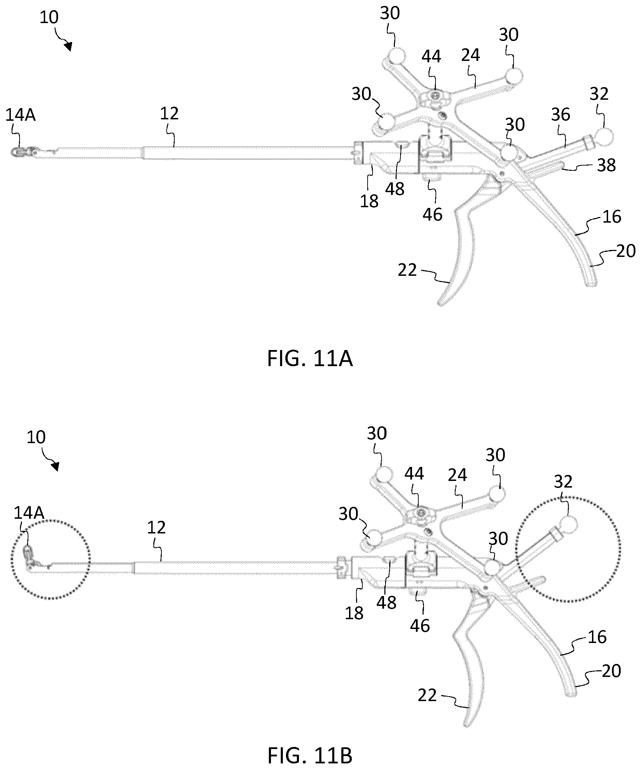

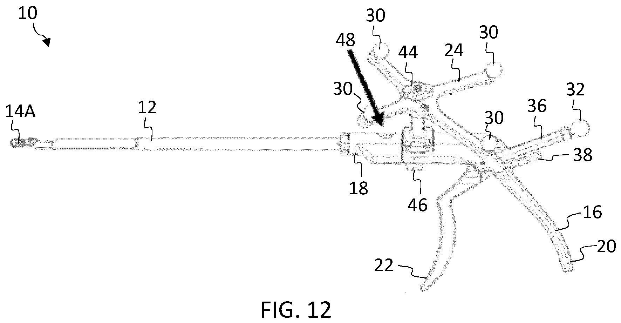

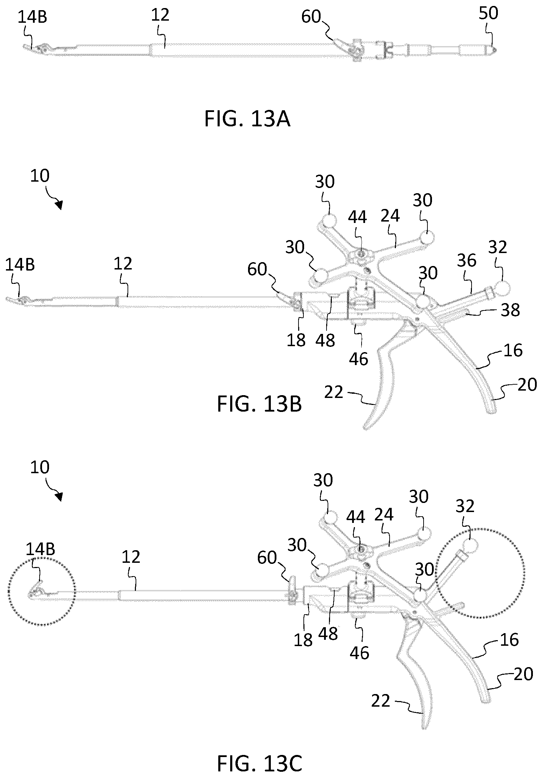

With this modular platform, different attachments 12 may be attached to the same handpiece 16. FIGS. 11A-13C depict a method of swapping one type of articulating tip 14A for another type of articulating tip 14B according to one embodiment. As shown in FIGS. 11A-11B, the instrument 10 may be assembled with a first type of articulating tip 14A, such as a curette having a sweep angle of up to 90°. The handpiece 16 may have a given translation or arc of movement for the tracking marker 32, which correlates to the pivotal motion of the articulating tip 14A. At any suitable time, the first attachment type may be replaced with a different attachment type. As best seen in FIG. 12, button 48 may be pressed to unlock and release the attachment shaft 12. The shaft 12 may be withdrawn from the barrel 18 of the handpiece 16. As shown in FIGS. 13A-13C, a new shaft 12 may be selected with a second type of articulating tip 14B, such as a curette having a sweep angle of up to 180°. The handpiece 16 may have a given translation or arc of movement for the tracking marker 32, which correlates to the movement of the new articulating tip 14B. The translation or arc of movement for tracking marker 32 may vary depending on the type and range of motion of articulating tip 14A, 14B. Thus, each tool may be tracked with the same primary array 24 that remains consistent, but with independent curve lengths (e.g., rotary translation) of the additional marker 32. This may be used to help differentiate tool types if the incorrect tool is selected in the workflow user interface on the robotic navigation system. The modular navigable platform may provide a more user friendly, economic, and customizable experience.

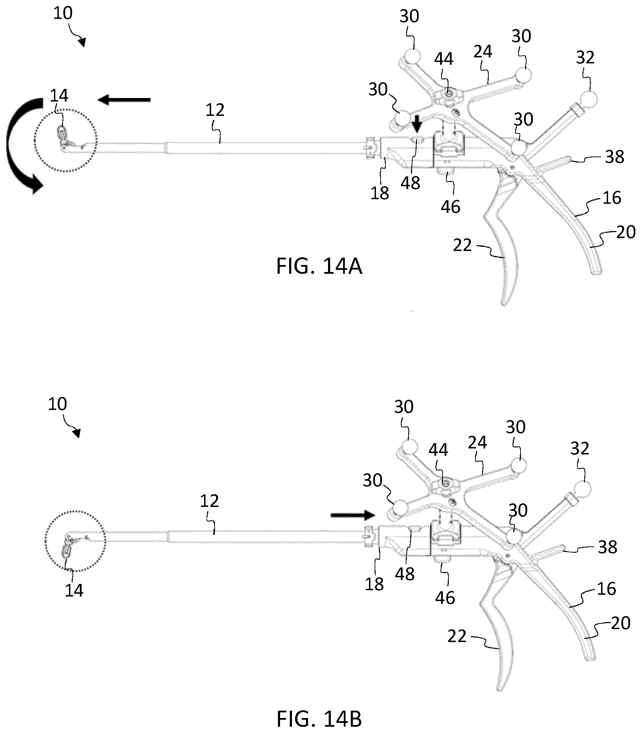

In addition to swappable attachments 12 and a flippable array 24, the tool attachment tip 14 may also be flipped 180° to enhance the surgeon's experience. As best seen in FIGS. 14A-14B, the release lock 48 may be pressed, the shaft 12 may be pulled out and the attachment shaft 12 may be rotated to reorient the articulating tip 14. The shaft 12 may be pushed back into the barrel 18 and relocked to the handpiece 16 in the inverse position. The ease of tip reorientation makes this platform user friendly, and re-orienting the attachment is as seamless as connecting a new modular attachment to the navigated handpiece 16.

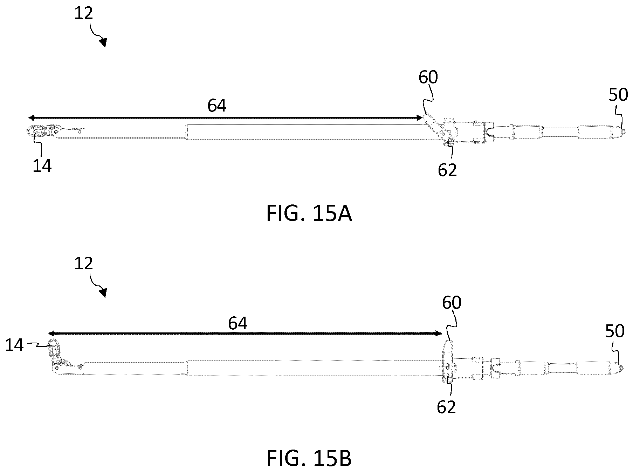

Turning now to FIGS. 15A-15B, the instrument 10 may include a built-in adjustable stop 60 on specific attachments. During a posterior discectomy, surgeons may be performing the discectomy and evacuation with minimal visualization and fluoroscopy imaging, creating a risk of surpassing the desired posterior-to-anterior depth with the possibility of breaking through the anterior longitudinal ligament (ALL) and impinging on the vascular organs. To reduce this risk while also allowing surgeons to meet the desired anterior depth, one or more depth controls may be put in place to maximize the discectomy. The robotic system 70, 100 may control the maximum depth, while the adjustable stop 60 allows for the articulating tool to remain at that max depth. The adjustable stop 60 may include a pivotable member, for example, which may be pinned to the shaft 12 or a connecting piece. The stop 60 may be located at a fixed location to set a fixed length 64 on the shaft 12 for the given articulating tip 14. Alternatively, the stop 60 may be configured to slide along the length of the tool shaft 12 to set a desired fixed length 64. For example, the stop 60 may have an internal mechanism, such as a spring-loaded pin or thumbscrew, to allow the stop 60 to move along the shaft 12 and when tightened, thereby secure the stop 60 at the desired location.

As best seen in FIGS. 15A-15B, the stop 60 may be set at fixed length 64 (e.g., 230mm) such that when the articulating tip 14 is rotated, the stop 60 pivots and maintains the same fixed length 64 (e.g., 230mm) between the distal-most point of the articulating tip 14 and the distal-most point of the stop 60. In FIG. 15A, the articulating tip 14 is shown in line with the longitudinal tool axis, and the stop 60 is tilted proximally toward the distal tip 14, thereby providing fixed length 64. In FIG. 15B, the articulating tip 14 is shown angled relative to the tool shaft 12, and the stop 60 is aligned generally perpendicular to the tool shaft 12, thereby maintaining the same fixed length 64. Thus, throughout the entire range of motion, the distance between the distal-most point of the tip 14 and distal-most point of the stop 60 remains constant. The stop 60 may help to ensure the articulating tip 14 maintains the prescribed depth limit. The built-in adjustment stop 60 may help to accomplish a thorough discectomy to improve interbody placement, increase graft delivered, and promote fusion, while minimizing risk and enhancing patient safety.

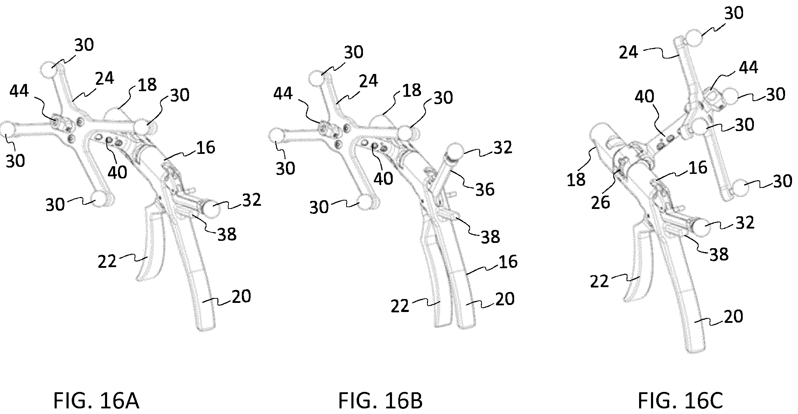

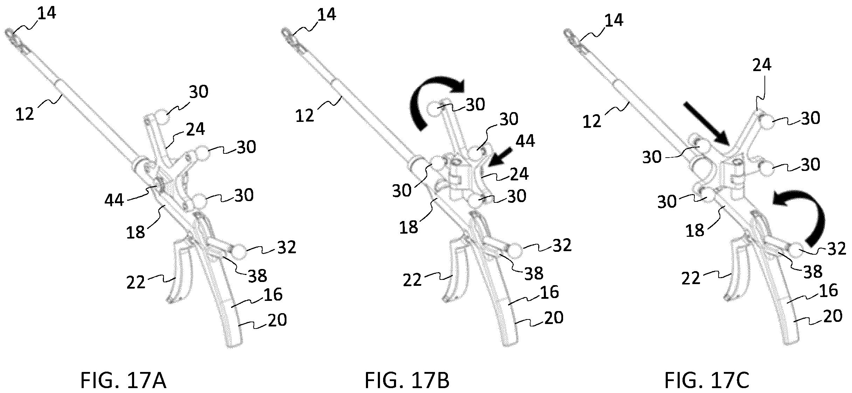

With further emphasis on FIGS. 16A-19B, the swappable array position may be compatible with extended reality (XR) systems. Extended reality is an all-encompassing term that augmented, virtual, and mixed realities may be summarized within. It may include the act of layering technological input elements over the landscape around us, in this case, in the operating (OR). The overlay can provide precise virtual information in real time to assist the surgeon with surgical tasks. Extended reality may allow surgeons to interact with preoperative plans, assess intraoperative navigation, and alter plans based on intraoperative feedback, to name a few capabilities. With this innovation that blends digital inputs overlaying into the real-world, there is a need to make this platform compatible with camera positions that may be located on the user (e.g., on a headset). In one embodiment, the instrument 10 may include an additional array position that directly faces the user (e.g., as shown in FIG. 17C). As best seen in FIGS. 17A-17C, the swappable array positions may include a standard left position, a standard right position, and an additional XR user position. The array 24 may be rotated such that the array 24 faces back toward the user and headset. The additional marker 32 also translates in view of the headset, which is captured by the system.

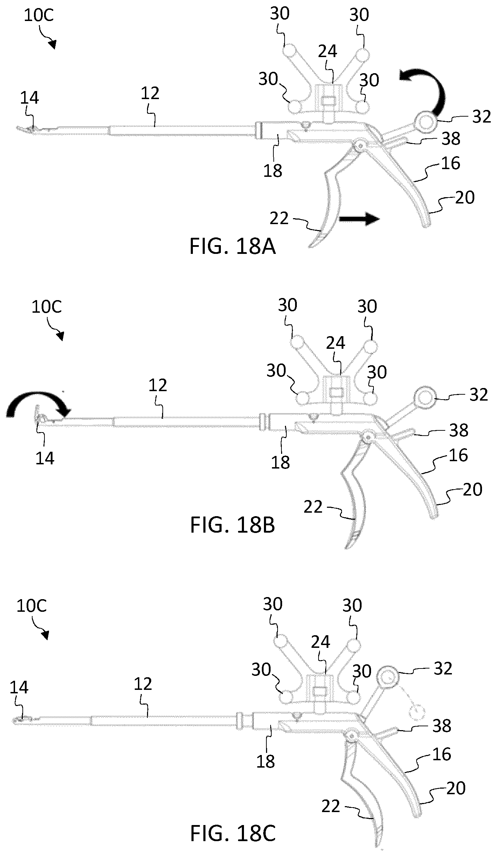

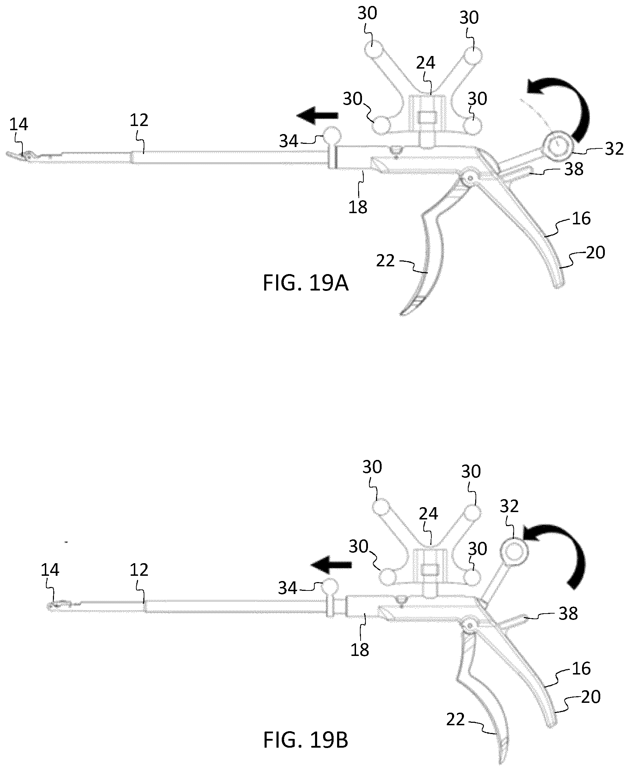

FIGS. 18A-18C show another example of articulating tool navigation with the pivotable array pattern and translating marker 32, which is compatible with extended reality systems. As described above for FIGS. 10A-10C, the modular design facilitates easy attachment and detachment of the tool shaft 12 while ensuring a stable and secure connection. Movement of the tip 14 may be tracked via tracking marker 32. As the moveable handle 22 is pulled toward the fixed handle 20, the force causes articulation of the tip 14. The tip 14 follows an articulating path, which matches or correlates to the translation path of the translating marker 32. FIGS. 19A-19B compare linear motion of marker 34 to rotary motion of marker 32, similar to as previously described for FIGS. 4A-4B.

In extended reality systems, duplicate markers may be removed from the field of view to not confuse marker visibility and simplify trackability. The tilted array 24 may split the difference between user-monitored cameras and independent cameras at the base of the table. The inline marker 32 allows for the translating marker to stay on the same arc rotation regardless of camera position in the operating room. This may help to improve visualization as extended reality compatibility may shift the location of camera systems in the operating room from standard, foot of the table locations to above the table (top-down view) and/or on the users headset in an effort to enhance tracking capabilities. Extended reality combability may work across a multitude of camera systems to improve the user experience. The instruments 10 may integrate seamlessly with a diverse range of visual input devices allowing for immersive visualization, thereby enhancing the interactive experience.

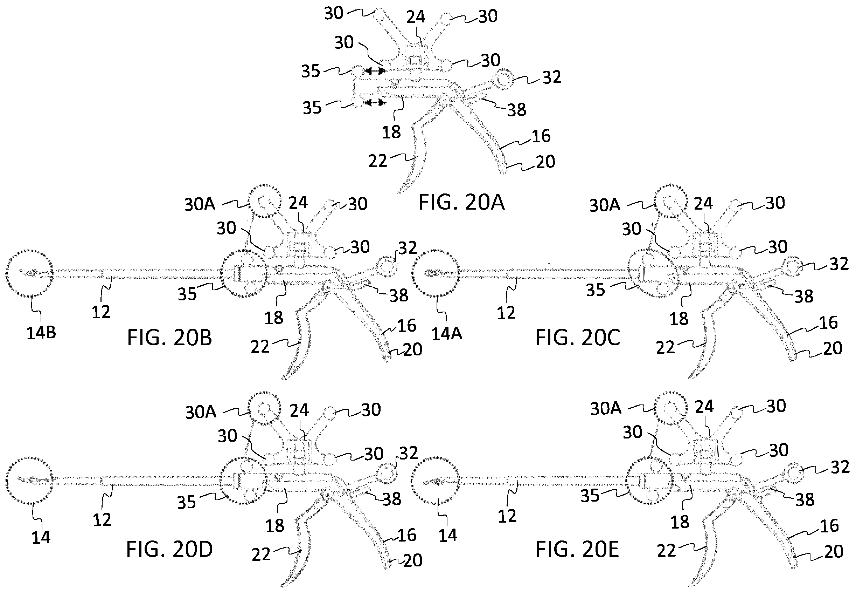

Turning now to FIGS. 20A-21B, the system may be configured to automate tool identification of various tools while streamlining the interchange of tool attachments 12 on the navigated handpiece 16. For tool navigation, specific array patterns rigidly connect to the tools, which are recognized and assigned in the software as a specific tool to display the profile, size, length, and orientation when the array 24 is observable by the camera system. With many tools utilized during a procedure, this may introduce complexity for the user. Although navigation was intended to reduce the cognitive load of surgeons, instead it may have the opposite effect due to the number of tools, arrays, and interaction with the system user interface. The modular platform may be configured to automate tool differentiation throughout the procedure, thereby reducing the number and complexity of tasks for the user. The differentiated tools may autonomously reduce the cognitive load on the user. By simplifying the swapping of tool attachments 12 on the independent navigated handpiece 16 and providing automatic tool identification, each tool attachment 12 may be recognized independently and automatically by the navigation system.

As a result of the modular platform, several different tool attachments 12 may utilize the same primary array pattern 24 to recognize tool location and orientation. This may be controlled through the software platform, for example, by manually selecting different tools that were previously verified over the course of the procedure through the user interface of the navigation system. In one embodiment, this step may be automated by the navigation system, for example, utilizing one or more additional fiducials or markers 35, which correlate to specific tool types or configurations. The automatic system may reduce manual inputs, thereby improving the user interface of the navigation system and experience of the user.

As shown in FIG. 20A, the additional tracking markers 35 may include a pair of differentiation markers 35. The differentiation markers 35 may be located on the handpiece 16 or on the tool attachment shaft 12. In one embodiment, the differentiation markers 35 may be located on the free end of the barrel 18 of the handpiece 16. The pair of markers 35 may be diametrically opposed on opposite sides of the barrel 18. One or both of the differentiation markers 35 may be configured to slide or translate, for example, along the longitudinal tool axis. The differentiation markers 35 may be capable of differentiating tool tips 14 and/or tip orientation or movement. The differentiation markers 35 may be actively translated to a new final position that corresponds with a given attachments tip and navigation.

In one embodiment, a pair of differentiation markers 35 may be provided to differentiate tools with a marker offset. In other words, the opposite markers 35 may be shifted or displaced relative to each other, thereby introducing an offset relative to one another. As best seen in FIG. 20B, the offset markers 35 may have a given position relative to each other and a primary marker 30A of the array 24 for a given tool tip 14B. In FIG. 10C, the offset markers 35 have an alternative positioned relative to one another and the primary marker 30A to signify a different tool tip 14A. The new corresponding marker positions 35 may include an increased distance between the offset markers 35 or any other suitable relationship. As such, differentiating tool-to-tool may be achieved through recognizing the fully altered marker locations.

With further emphasis on FIGS. 20D and 20E, the position or orientation of the articulating tip 14 may also be automatically discerned by the navigation system. As shown in FIG. 20D, the tool tip 14 may have an upward-facing orientation, which is identified by the offset markers 35 having a given position relative to each other and the primary marker 30A of the array 24. In FIG. 20E, the tool tip 14 is reversed and has a downward-facing orientation. The inverse tool tip 14 is identifiable by the inverse offset markers 35. As such, differentiating tip-to-tip may be through inversely related marker locations. It will be appreciated that the number, location, and movement of the differentiation markers may be selected to optimize automatic tracking and identification.

The differentiation markers 35 allow the primary array 24 to have a family of tools it works with, plus the marker locations of those tools. The automatic tool identification places more emphasis on the camera system and user interface to guide the user through the case rather than manual inputs from the user. The automatic tool differentiation system may help to reduce the cognitive load and monotonous task of selecting new tools throughout the procedure.

The capability and complexity of the system to navigate and differentiate amongst the tools allocated to the set may also be correlated to the machine learning model, for example, by stressing the importance of tracking where, when, which and for how long each tool is used throughout the surgery. AI models may also be used to both improve navigation and differentiate tools based on instrumentation specific actuation. This allows camera systems to identify tools and the degree of articulation based on unique geometry, profile, and landmarks that are implemented in the design of the tool, eliminating the requirement to include markers to identify instrumentation. Elimination of tracking markers may greatly reduce cost, weight, and/or software/hardware complexity.

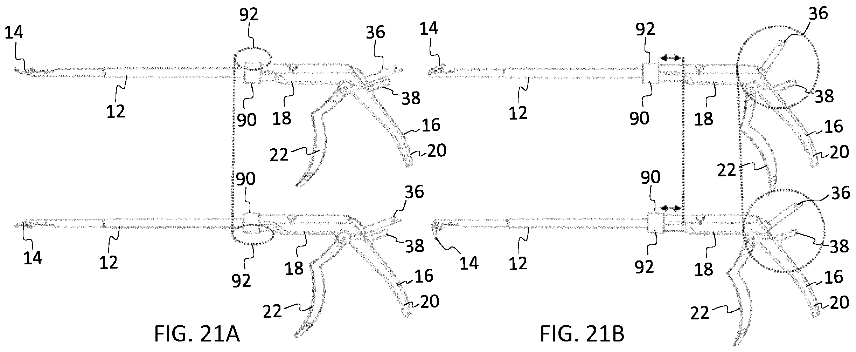

With further emphasis on FIGS. 21A-21B, the instrumentation may include a translating sleeve 90 with an identifying area or landmark 92, which may be observable, for example, by a machine vision camera or other advanced sensing technologies. The landmark 92 on the sleeve 90 may include a shape, such as a recess, indent, groove, or other identifying feature. As best seen in FIG. 21A, the system may automatically discern the orientation of the tip 14 (e.g., upward-facing or downward-facing) based on the corresponding location of the landmark 92. As best seen in FIG. 21B, the system may automatically track the articulation of the tip 14 based on the position of the translating sleeve 90 relative to the static barrel 18 of the handpiece 16. In addition, or alternatively, the system may track the relative position of the translating post 36 relative to the static post 38. Thus, tip orientation and location as well as instrumentation movement may be utilized to navigate tip position and articulation.

The inclusion of machine vision camera or other advanced sensing technology in the operating room may allow for removal of tracking markers from instrumentation to reduce instrumentations size, weight and complexity. This instrumentation includes the same modularity while incorporating innovative solutions to improve the users experience and increase surgeon adoption by providing a platform that works across several navigating systems. As navigation systems develop, camera systems may be located in new positions relative to the operating table to enhance the visualization of fiducials and/or instrument specific landmarks. This modular system will remain compatible with these industry upgrades suggesting that as camera systems develop and improve, so will the intraoperative navigation for articulating tools.

Although several embodiments of the invention have been disclosed in the foregoing specification, it is understood that many modifications and other embodiments of the invention will come to mind to which the invention pertains, having the benefit of the teaching presented in the foregoing description and associated drawings. It is thus understood that the invention is not limited to the specific embodiments disclosed hereinabove, and that many modifications and other embodiments are intended to be included within the scope of the appended claims. It is further envisioned that features from one embodiment may be combined or used with the features from a different embodiment described herein. Moreover, although specific terms are employed herein, as well as in the claims which follow, they are used only in a generic and descriptive sense, and not for the purposes of limiting the described invention, nor the claims which follow. The entire disclosure of each patent and publication cited herein is incorporated by reference in its entirety, as if each such patent or publication were individually incorporated by reference herein. Various features and advantages of the invention are set forth in the following claims.

The invention could be defined inter alia by the following examples:
1. A method of performing a discectomy, the method comprising:
   (a) providing a database model based on existing patient data with discectomy data;
   (b) conducting preoperative imaging and planning for a given patient;
   (c) obtaining a customized surgical plan from the database model including an approach, location, and degree of discectomy for the given patient;
   (d) performing a discectomy based on guidance from the customized surgical plan and collecting intraoperative data;
   (e) conducting a postoperative analysis after the discectomy and collecting postoperative data; and
   (f) updating the database model based on the intraoperative and postoperative data.
2. The method of Example 1, wherein the discectomy is conducted with a navigated articulating discectomy instrument.
3. The method of Example 1, wherein for a non-fusion procedure, the discectomy targets an identified surgical level and identifies soft tissue to be removed based on a volumetric heatmap.
4. The method of Example 1, wherein for a fusion procedure, the discectomy targets an identified surgical level, selects a surgical approach, identifies soft tissue to be removed based on a volumetric heatmap, and prepares the disc space for insertion of an interbody implant.
5. The method of Example 4 further comprising modifying the volumetric heatmap based on intended placement of the interbody implant.
6. The method of Example 1, wherein the database model incorporates artificial intelligence to enhance database functionality, data analysis, and predictions.
7. The method of Example 1, wherein the database model is incorporated into software of an on-board computer for a surgical robotic and navigation system.
8. A method of performing a spinal decompression of a patient, the method comprising:
   obtaining a customized discectomy plan from a database model with existing discectomy data based on patient specific parameters for the patient; inserting a navigated discectomy instrument into the disc space, the instrument having an articulating distal tip and a corresponding tracking marker configured to track movement of the articulating distal tip when articulated; tracking location, orientation, and movement of the articulating distal tip by a navigation system to perform a discectomy based on the customized discectomy plan; and collecting data throughout the discectomy and updating the database model with the newly collected data.
9. The method of Example 8, wherein the tracking marker follows an arc to finely track the tip location as it articulates.
10. The method of Example 8, wherein a path of the tracking marker is constant for a life cycle of the tip movement.
11. The method of Example 8, wherein the tracking marker is coupled to a moveable handle, which actuates the articulating distal tip.
12. The method of Example 8, wherein the navigated discectomy instrument is navigated with a tracking array having multiple orientations to maintain line of sight with the navigation system.
13. The method of Example 12, wherein the tracking array is rotatable to discrete left, right, and top orientations.
14. The method of Example 8 further comprising swapping one articulating distal tip and installing another type of articulating distal tip on the navigated discectomy instrument.
15. The method of Example 8 further comprising reorienting the articulating distal tip by releasing a shaft and rotating the shaft to reorient the tip 180 degrees from its initial position.
16. A system for performing a discectomy, the system comprising: a surgical robotic and navigation system having an on-board computer with software executed by one or more processing units, and storing and executing an existing database model with existing discectomy data; and a navigated discectomy instrument having a tracking array and an articulating distal tip with a corresponding tracking marker configured to track movement of the articulating distal tip when articulated, wherein the surgical robotic and navigation system provides customized guidance to a surgeon during a discectomy, and location, orientation, and movement of the articulating distal tip is trackable by the surgical robotic and navigation system.
17. The system of Example 16, wherein the navigated discectomy instrument includes a modular handpiece for receiving a variety of tool attachments with different types of articulating distal tips.
18. The system of Example 16, wherein the navigated discectomy instrument includes a handpiece with a barrel for receiving a shaft, a fixed handle, and a moveable handle pivotably coupled to the fixed handle configured to articulate the articulating distal tip.
19. The system of Example 18, wherein the barrel includes a rotatable collar configured to rotate about a longitudinal tool axis of the barrel to orient the tracking array, and the rotatable collar includes a rotational lock configured to secure and lock the tracking array into place when the array is optimally positioned.
20. The system of Example 18, wherein the shaft includes a depth control including a pivotable stop such that when the articulating distal tip is rotated, the stop pivots and maintains a fixed length between a distal-most point of the articulating distal tip and a distal-most point of the stop.

## Claims

1. A system for performing a discectomy, the system comprising:
a surgical robotic and navigation system having an on-board computer with software executed by one or more processing units, and storing and executing an existing database model with existing discectomy data; and
a navigated discectomy instrument having a tracking array and an articulating distal tip with a corresponding tracking marker configured to track movement of the articulating distal tip when articulated,
wherein the surgical robotic and navigation system provides customized guidance to a surgeon during a discectomy, and location, orientation, and movement of the articulating distal tip is trackable by the surgical robotic and navigation system.

2. The system of claim 1, wherein the navigated discectomy instrument includes a modular handpiece for receiving a variety of tool attachments with different types of articulating distal tips.

3. The system of claim 1 or 2, wherein the navigated discectomy instrument includes a handpiece with a barrel for receiving a shaft, a fixed handle, and a moveable handle pivotably coupled to the fixed handle configured to articulate the articulating distal tip.

4. The system of claim 3, wherein the barrel includes a rotatable collar configured to rotate about a longitudinal tool axis of the barrel to orient the tracking array, and the rotatable collar includes a rotational lock configured to secure and lock the tracking array into place when the array is optimally positioned.

5. The system of claim 3 or 4, wherein the shaft includes a depth control including a pivotable stop such that when the articulating distal tip is rotated, the stop pivots and maintains a fixed length between a distal-most point of the articulating distal tip and a distal-most point of the stop.

6. The system of any one of the preceding claims wherein the system further comprises a unit suitable for generating an auto-plan, the autoplan generating preferably a volumetric heatmap that isolates the area to be operated, the heatmap including preferably the segmented disc level and soft tissue targeted for removal.

7. The system of the preceding claim wherein the volumetric heatmap comprises a simulated image of the intervertebral disc and/or disc space including access corridors, areas to target tissue removal, areas to target endplate preparation.

8. The system of the claim 7 wherein the simulated image provides a detailed three-dimensional representation of the disc, highlighting areas to be targeted for tissue removal.

9. The system of any one of claims 6 to 8 wherein the heatmap includes gradients of different colors to represent different ranges of data values.

10. The system of any one of claims 6 to 9 and further comprising a manipulating and examnation unit for manipulating the heatmap and examining the heatmap from various perspectives to offer insights on multiple angles and depths to assess and plan the surgery..

11. The system of any one of the preceding claims wherein the system comprises at least one of the following:
a. an artificial intelligence (AI) model offering customized recommendations to the surgeon;
b. an in-depth preoperative analysis and plan of soft tissue removal that breaks down specific areas of discectomy;
c. A control unit for navigating, cataloging, and differentiating different surgical instruments;
d. depth controls to enhance surgical precision and safety to ensure the tools do not penetrate deeper than necessary during the procedure;
e. connection unit for connecting the system to extended reality (XR), which may allow the surgeon to interact with preoperative plans, assess intraoperative navigation, and alter plans based on intraoperative feedback.
f. connection unit for connecting the system to a machine vision system to track the instrument, without markers, throughout the discectomy procedure.
